(19) Europäisches Patentamt
European Patent Office
Office européen des brevets

(11) **EP 4 009 960 B1**

(12) **EUROPEAN PATENT SPECIFICATION**

(45) Date of publication and mention
of the grant of the patent:
**04.06.2025 Bulletin 2025/23**

(21) Application number: **20751156.9**

(22) Date of filing: **07.08.2020**

(51) International Patent Classification (IPC):
*A61K 9/20* (2006.01)          *A61K 47/14* (2017.01)

(52) Cooperative Patent Classification (CPC):
**A61K 9/2054; A61K 9/2009; A61K 9/2013;
A61K 9/2018; A61K 31/155; A61K 36/81;**
A61J 3/10

(86) International application number:
**PCT/EP2020/072220**

(87) International publication number:
**WO 2021/023850 (11.02.2021 Gazette 2021/06)**

(54) **METHOD OF PREPARING A SOLID DOSAGE FORM AND A BINDER**

VERFAHREN ZUR HERSTELLUNG EINER FESTEN DARREICHUNGSFORM UND EINES
BINDEMITTELS

PROCÉDÉ DE PRÉPARATION D'UNE FORME POSOLOGIQUE SOLIDE ET D'UN LIANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.08.2019 IN 201941032098
10.10.2019 EP 19202430**

(43) Date of publication of application:
**15.06.2022 Bulletin 2022/24**

(73) Proprietor: **Evonik Operations GmbH
45128 Essen (DE)**

(72) Inventors:
• **GUHA, Ashish
Mumbai (IN)**
• **JAIN, Vinay
Mumbai 400004 (IN)**
• **JOSHI, Shraddha
Airoli Thane 400708 (IN)**
• **HERBEAUX, Jean-Luc
2750-225 Cascais (PT)**

(74) Representative: **Evonik Patent Association
c/o Evonik Industries AG
IP Management
Postcode 84/339
Rodenbacher Chaussee 4
63457 Hanau (DE)**

(56) References cited:
WO-A1-2017/202942      WO-A1-2019/034698
US-A1- 2010 272 792      US-A1- 2014 274 987

**Description**

The present invention is related to a method of preparing a solid dosage form, comprising the steps of: a. preparing a binder consisting of at least one polyunsaturated fatty acid salt; b. adding the binder and ingredients for the solid dosage form to a mixer; c. optionally carrying out one or more of the following steps: granulation, drying and sizing, d. blending the contents of the mixer; and e. compressing or slugging the blended contents to produce a solid dosage form wherein the binding parameter (BP) for the solid dosage form is at least 2 and is determined by: $BP = H/C$, wherein H is the tablet breaking force in Newton (N) and C is the compression force in kilo Newton (kN).

Binder excipients are formulated to act as an adhesive to "bind together" powders, granules and other dry ingredients to impart to the product the necessary mechanical strength. They can also give volume to low active dose tablets. Whether produced using wet granulation or dry granulation, roll compaction or even direct compression, the binder ensures integrity and is crucial to stability across the formulation lifetime.

Binders are usually saccharides and their derivatives, such as the disaccharides: sucrose, lactose; polysaccharides and their derivatives, such as starches, cellulose or modified cellulose such as microcrystalline cellulose and cellulose ethers such as hydroxypropyl cellulose (HPC); sugar alcohols such as xylitol, sorbitol or mannitol; Proteins, such as gelatin; synthetic polymers, such as polyvinylpyrrolidone (PVP) or polyethylene glycol (PEG).

Binders are classified according to their application: Solution binders are dissolved in a solvent (for example water or alcohol can be used in wet granulation processes). Examples include gelatin, cellulose, cellulose derivatives, polyvinylpyrrolidone, starch, sucrose and polyethylene glycol. Dry binders are added to the powder blend, either after a wet granulation step, or as part of a direct powder compression (DC) formula. Examples include cellulose, methyl cellulose, polyvinylpyrrolidone and polyethylene glycol.

A review article entitled "A flexible technology for modified-release drugs: Multiple-unit pellet system (MUPS)" discusses the phenomena, mechanisms and challenges including film cracking during compression. It also highlights the requirement of a flexible film coat to avoid damage to coated pellets during compression (Abdul et al., Journal of Controlled Release 147 (2019) 2-16).

WO 2019/034698 A1 discloses a tablet comprising a composition containing one or more omega-3 fatty acid amino acid salts, binders and lubricants with fast disintegration and a process for producing such tablets. US 2014/274987 A1 discloses a solid pharmaceutical composition comprising nicotinic acid and eicosapentaenoic acid or derivative thereof. The composition contains not more than 10% by weight docosahexaenoic acid. Also a process for producing such composition is disclosed. WO 2017/202942 A1 discloses a pharmaceutical composition comprising estradiol in a solid dosage form and linoleic acid and/or arachidonic acid and a process for preparing such dosage form. US 2010/272792 A1 discloses a tablet comprising 100 to 300 mg dry docosahexaenoic acid and an additive, selected from elemental iron, calcium ascorbate, silicon dioxide, calcium carbonate, chelated magnesium, chelated copper and combinations thereof, in an amount sufficient to stabilize the docosahexaenoic acid from oxidative degradation. None of these documents disclose the use of a preparation comprising at least one salt of a polyunsaturated fatty acid as binder in tabletting applications for compression of solid components.

Problem: Compression of tablets on industrial scale high speed compression machines requires high compressibility at wider compression forces but still yielding tablets with high strength and low friability. Most of the diluents, carriers or binders provide enough strength to the tablets but usually within narrow range of compression forces. In such formulations, lower compression forces yield tablets with poor strength, while those prepared with higher than optimal forces, tend to pose problems like capping. Consistent running of machines at high compression forces leads to early wear & tear, along with higher energy consumption. In order to avoid this problem, if such machines are run at lower limit of compression forces, there is a risk of quality failure in some of the tablets related to its strength. Thus, there is a need to develop suitable processes wherein acceptable tablet properties can be achieved at wider compression forces.

Similarly, manufacturing of specialized tablets such as MUPS are especially sensitive to changes in compression forces normally encountered during manufacturing, leading to variation in quality, induced by issues with integrity of embedded coated particles or tablets itself. Additionally, it is also required to have acceptable cushioning for functional particles to avoid crushing and loss of functionality.

Thus, it is required to develop easy to process formulations encompassing all the characteristics mentioned above. Furthermore, it is required to develop such formulations free from magnesium stearate.

Solution: It was surprisingly found that when solid powdered fatty acid salts are used as binder in the tablet formulations, they provide exceptionally good tablet characteristics at very low compression forces inherently suitable for high speed compression machines. These formulations containing fatty acid salts in the concentration range of 5 to 50 % w/w could be formulated using widely used tableting technologies including direct compression and wet granulation.

Additionally, it was found that such formulations can provide good cushioning to the sensitive components of the dosage forms, such as for instance in case of multiple unit particulate system (MUPS) tablets. Furthermore, it is preferable to use amino acid salts of polyunsaturated fatty acids as binder in absence of magnesium stearate in order to obtain a superior product.

The invention provides alternate formulations for nutraceutical and pharmaceutical tablets, using fatty acid salts as carrier and binder providing unique manufacturing advantages like high speed processing on commercial machines while avoiding health issues associated with ingredients such as magnesium stearate.

The present invention is related to a method of preparing a solid dosage form, comprising the steps of:

a. preparing a binder consisting of at least one polyunsaturated fatty acid salt;
b. adding the binder and further ingredients to a mixer;
c. optionally carrying out one or more of the following steps: granulation, drying and sizing,
d. blending the contents of the mixer;
e. compressing or slugging the blended contents to produce a solid dosage form,

wherein the binding parameter (BP) for the solid dosage form is at least 2 and is determined by:

$$BP = \frac{H}{C}$$

wherein H is the tablet breaking force in Newton (N) and C is the compression force in kilo Newton (kN).

A good binder is the one which provides good strength to the tablet at low compression force, therefore, the binding parameter of a composition is a suitable parameter to determine binding capacity of a certain substance.

The binding parameter is used without units. However, it may be used with the unit, which is N / kN. According to the present invention, the binding factor of the solid dosage form is at least 2. In preferred configurations, the binding factor is at least 2.2, or at least 2.3, or at least 2.4, or at least 2.5, or at least 3.

With this method, solid dosage forms can easily be prepared which are suitable for direct compression and without the need of further known binders, due to the surprising binding effect of the polyunsaturated fatty acid salts.

In capsule filling operation, the powder is filled into capsules usually after forming a slug in both, dosator and tamping based capsule filling machines. Similarly, in order to produce granules of higher density and flow, the powder is compressed between the two rollers or punches to form slugs, which are further broken down into smaller particles and processed further into different dosage forms. Those processes are referred to as slugging according to the present invention.

In a preferred embodiment, the friability of the solid dosage form is 5% or less, preferably 3% or less, more preferably 1% or less. Friability is the tendency for a tablet to chip, crumble or break following compression.

Numerous health benefits have been correlated with the supplemental intake of polyunsaturated fatty acids (PUFAs) by an extensive body of evidence gathered over the course of the past several decades. Prevention of cardiovascular disease and reducing the symptoms of inflammatory conditions are amongst the most prominent examples, however, preventing the promotion and progression stages of some types of cancer, reducing blood pressure and blood cholesterol as well as positive effects in the treatment of depression and schizophrenia, Alzheimer's disease, dyslexia, and attention-deficit or hyperactivity disorder, amongst others, have been reported as well. Furthermore, because some PUFAs are considered to be essential for the development of brain, nervous system and eye, nowadays routinely, infant nutrition is supplemented with specific PUFAs.

In the context of the present invention the term PUFA is used interchangeably with the term polyunsaturated fatty acid and defined as follows: Fatty acids are classified based on the length and saturation characteristics of the carbon chain. Short chain fatty acids have 2 to about 6 carbons and are typically saturated. Medium chain fatty acids have from about 6 to about 14 carbons and are also typically saturated. Long chain fatty acids have from 16 to 24 or more carbons and may be saturated or unsaturated. In longer chain fatty acids there may be one or more points of unsaturation, giving rise to the terms "monounsaturated" and "polyunsaturated," respectively. In the context of the present invention long chain polyunsaturated fatty acids having 20 or more carbon atoms are designated as polyunsaturated fatty acids or PUFAs.

PUFAs are categorized according to the number and position of double bonds in the fatty acids according to well established nomenclature. There are two main series or families of LC-PUFAs, depending on the position of the double bond closest to the methyl end of the fatty acid: The omega-3 series contains a double bond at the third carbon, while the omega-6 series has no double bond until the sixth carbon. Thus, docosahexaenoic acid (DHA) has a chain length of 22 carbons with 6 double bonds beginning with the third carbon from the methyl end and is designated "22:6 n-3" (all-cis-4,7,10,13,16,19-docosahexaenoic acid). Another important omega-3 PUFA is eicosapentaenoic acid (EPA) which is designated "20:5 n-3" (all-cis-5,8,11,14,17-eicosapentaenoic acid). An important omega-6 PUFA is arachidonic acid (ARA) which is designated "20:4 n-6" (all-cis-5,8,11,14-eicosatetraenoic acid).

Other omega-3 PUFAs include: Eicosatrienoic acid (ETE) 20:3 (n-3) (all-cis-11,14,17-eicosatrienoic acid), Eicosatetraenoic acid (ETA) 20:4 (n-3) (all-cis-8,11,14,17-eicosatetraenoic acid), Heneicosapentaenoic acid (HPA) 21:5 (n-3) (all-cis-6,9,12,15,18-heneicosapentaenoic acid), Docosapentaenoic acid (Clupanodonic acid) (DPA) 22:5 (n-3) (all-cis-7,10,13,16,19-docosapentaenoic acid), Tetracosapentaenoic acid 24:5 (n-3) (all-cis-9,12,15,18,21-tetracosapentaenoic acid), Tetracosahexaenoic acid (Nisinic acid) 24:6 (n-3) (all-cis-6,9,12,15,18,21-tetracosahexaenoic acid).

Other omega-6 PUFAs include: Eicosadienoic acid 20:2 (n-6) (all-cis-11,14-eicosadienoic acid), Dihomogamma-linolenic acid (DGLA) 20:3 (n-6) (all-cis-8,11,14-eicosatrienoic acid), Docosadienoic acid 22:2 (n-6) (all-cis-13,16-docosadienoic acid), Adrenic acid 22:4 (n-6) (all-cis-7,10,13,16-docosatetraenoic acid), Docosapentaenoic acid (Osbond acid) 22:5 (n-6) (all-cis-4,7,10,13,16-docosapentaenoic acid), Tetracosatetraenoic acid 24:4 (n-6) (all-cis-9,12,15,18-tetracosatetraenoic acid), Tetracosapentaenoic acid 24:5 (n-6) (all-cis-6,9,12,15,18-tetracosapentaenoic acid).

Preferred omega-3 PUFAs used in the embodiments of the present invention are docosahexaenoic acid (DHA) and eicosapentaenoic acid (EPA).

In a further preferred configuration, the omega-3 or omega-6 fatty acid salts have an organic counter ion selected from lysine, arginine, ornithine, choline and mixtures of the same.

In a preferred embodiment of the present invention, when the solid dosage form is a tablet, the blended contents are compressed in a tableting machine and the ejection force of the tableting machine is not more than 150N, preferably not more than 130N, more preferably not more than 120N, most preferably between 50N and 120N.

It is particularly preferred when the mean particle size of the binder before mixing is between 2 μm and 600 μm.

In an advantageous configuration of the present invention, the binder is prepared by

admixing aqueous, aqueous-alcoholic or alcoholic solutions of a composition comprising at least one polyunsaturated omega-3 fatty acid or omega-6 fatty acid component and a composition containing a basic organic acid selected from lysine, arginine, ornithine, choline or at least counter ion selected from magnesium ($Mg^{2+}$) and potassium ($K^+$) and mixtures of the same, and

subjecting resulting admixture to spray drying conditions or an extruder-based process subsequently, thus forming a solid product composition comprising at least one salt of a cation derived from the basic amino acid or magnesium ($Mg^{2+}$) or potassium ($K^+$) with an anion derived from a polyunsaturated omega-3 fatty acid or omega-6 fatty acid.

Recently, a technology has been described to stabilize EPA/DHA free fatty acids with amino acids resulting in solid and somewhat inert salts of EPA/DHA that can be introduced into e.g. food or supplement preparations. WO2016102323A1 describes compositions comprising polyunsaturated omega-3 fatty acid salts that can be stabilized against oxidation.

Compositions comprising polyunsaturated fatty acids that can be stabilized against oxidation may be obtained from any suitable source material which, additionally, may have been processed by any suitable method of processing such source material. Typical source materials include any part of fish carcass, vegetables and other plants as well as material derived from microbial and/or algal fermentation. Typically, such material further contains substantial amounts of other naturally occurring fatty acids. Typical methods of processing such source materials may include steps for obtaining crude oils such as extraction and separation of the source material, as well as steps for refining crude oils such as settling and degumming, de-acidification, bleaching, and deodorization, and further steps for producing PUFA-concentrates from refined oils such as de-acidification, transesterification, concentration, and deodorization (cf. e.g. EFSA Scientific Opinion on Fish oil for Human Consumption). Any processing of source materials may further include steps for at least partially transforming PUFA-esters into the corresponding free PUFAs or inorganic salts thereof.

Preferred compositions comprising PUFAs that can be stabilized against oxidation by the process of the present invention can be obtained from compositions mainly consisting of PUFA esters and other naturally occurring fatty acids by cleavage of the ester bonds and subsequent removal of the alcohols previously bound as esters. Preferably, ester cleavage is performed under basic conditions. Methods for ester cleavage are well known in the art.

According to the present invention, the spray drying conditions comprise a pure spray drying or a spray granulation process, or continuous spray granulation.

Salts of lysine with polyunsaturated fatty acids per se are known in the art (cf. EP 0734373 B1), and were described as "very thick transparent oils, which transform into solids of waxy appearance and consistency at low temperatures" (cf. EP 0734373 B1, page 1, lines 47 to 48). However, salts of PUFAs can be obtained via spray drying conditions as described in WO2016102323A1 and WO2016102316A1.

The present invention is also directed to the use of a preparation comprising at least one polyunsaturated fatty acid salt comprising at least one omega-3 or omega-6 fatty acid salt selected from eicosapentaenoic acid (EPA), docosahexaenoic acid (DHA), arachidonic acid (ARA), alpha linolenic acid, stearidonic acid, eicosatetraenoic acid, docosapentaenoic acid, linoleic acid, γ-linolenic acid as binder in tableting applications for compression of solid components.

In a preferred configuration, the omega-3 fatty acid component is selected from EPA or DHA. In a further preferred configuration, the omega-3 or omega-6 fatty acid salts have an organic counter ion selected from lysine, arginine, ornithine, choline or magnesium ($Mg^{2+}$) or potassium ($K^+$) and mixtures of the same.

In a preferred embodiment, the amount of polyunsaturated fatty acid is 65 weight-% or less, preferably 60 weight-% or less, more preferably between 40 and 55 weight-% with respect to the total weight of polyunsaturated fatty acid salt.

In a preferred embodiment, the amount of polyunsaturated fatty acid salt in the tableting composition is 70 weight-% or less, preferably 40 weight-% or less, more preferably between 5 and 40 weight-%.

## Working examples:

### Methods

1) Tablet friability test: Friability is the tendency for a tablet to chip, crumble or break following compression. This tendency is normally confined to uncoated tablets and surfaces during handling or subsequent storage. Friability test was carried out as per method described in USP chapter <1216>.

For tablets with a unit mass equal to or less than 650 mg, a sample of whole tablets corresponding to 6.5 g was taken. For tablets with a unit mass of more than 650 mg, a sample of 10 whole tablets was taken. The tablets were carefully dedusted prior to testing and were then weighted and placed in the drum. The drum was rotated 100 times and the tablets were removed afterwards. Loose dust was removed from the tablets and they were subsequently weighted. Generally, the test is run once. If obviously cracked, cleaved, or broken tablets are present after tumbling, the sample fails the test. If the results are doubtful or if the weight loss is greater than the targeted value, the test was repeated twice and the mean of the three tests was determined.

A maximum mean weight loss from the samples of not more than 1.0 % is considered acceptable for most products. For specialized dosage forms e.g. MUPS tablets, effervescent tablets and chewable tablets may have different specifications as far as friability is concerned.

2) Binding parameter (BP): Apart from friability, binding property of a formulation was also determined by calculating binding parameter as a function of the two important factors; tablet breaking force and compression force. A good binder is the one which provides good strength to the tablet at low compression force. For calculation of binding parameter BP following equation was used:

$$BP = \frac{H}{C}$$

wherein H is the tablet breaking force in Newton (N) and C is the compression force in kilo Newton (kN).

(a) Tablet breaking force (H): Tablet breaking force was measured by test procedures as described in USP general chapter <1217> using a modern tester employ mechanical drives, strain gauge-based load cells for force measurements, and electronic signal processing. The tablet was placed between the platens and the test was performed. After breaking of the tablet, the results were recorded as tablet breaking force in newton (N).

(b) Compression force (C): Compression force of tablets was measured from the compression machine equipped with sensors to measure forces applied for compression of tablets during tablet manufacturing. It is recorded in kilo newton (kN).

Acceptance criteria for binding parameter: For optimal binding property of a binder, the binding parameter (BP) should be more than 2.

### Polyunsaturated fatty acid compositions

In the examples for the present invention, different polyunsaturated fatty acid compositions were used. Different omega-3 fatty acid salts having an organic counter ion selected from the basic amino acids lysine and arginine were prepared. The omega-3 fatty acids Eicosapentaenoic acid (C20:5w3c) (EPA) and Docosahexaenoic acid (C22:6w3c) (DHA) are present in a ratio of around 2:1 (ratio EPA : DHA). The salts were prepared by spray granulation as described in WO2016102323A1.

The omega-3 lysine salt (omega-3-lys) contains around 32 weight-% of L-lysine and around 65 weight-% of polyunsaturated fatty acids (AvailOm®, Evonik Nutrition and Care GmbH, Germany). The major polyunsaturated fatty acids in the composition are the omega-3 fatty acids Eicosapentaenoic acid (C20:5w3c) (EPA) and Docosahexaenoic acid (C22:6w3c) (DHA), summing up to around 58 weight-% of the composition. The composition also contains minor amounts of Docosaenoic acid isomer (incl. erucic acid) (C22:1), Docosapentaenoic acid (C22:5w3c) and of the omega-6 fatty acids Arachidonic acid (C20:4w6) and Docosatetraenoic acid (C22:4w6c).

The omega-3 arginine salt (omega-3-arg) contains around 35 weight-% of L-arginine and around 64 weight-% of polyunsaturated fatty acids. The major polyunsaturated fatty acids in the composition are the omega-3 fatty acids Eicosapentaenoic acid (C20:5w3c) (EPA) and Docosahexaenoic acid (C22:6w3c) (DHA), summing up to around 49 weight-% of the composition. The composition also contains minor amounts of Docosaenoic acid isomer (incl. erucic acid) (C22:1), Docosapentaenoic acid (C22:5w3c) and of the omega-6 fatty acids Arachidonic acid (C20:4w6) and Docosatetraenoic acid (C22:4w6c).

The omega-3 ornithine salt (omega-3-orn) contains around 29 weight-% of L-ornithine and around 70 weight-% of polyunsaturated fatty acids. The major polyunsaturated fatty acids in the composition are the omega-3 fatty acids Eicosapentaenoic acid (C20:5w3c) (EPA) and Docosahexaenoic acid (C22:6w3c) (DHA), summing up to around 54 weight-% of the composition. The composition also contains minor amounts of Docosaenoic acid isomer (incl. erucic acid) (C22:1), Docosapentaenoic acid (C22:5w3c) and of the omega-6 fatty acids Arachidonic acid (C20:4w6) and Docosatetraenoic acid (C22:4w6c).

The $Mg^{2+}$ salts of omega-3 were prepared by kneading as described in WO2017202935A1 using the PUFA composition described above.

## A) Omega-3 amino acid salt as binder in Metformin tablets

Metformin tablets were prepared without addition of a binder (comparative examples C-1 and C-2) and with addition of an omega-3 lysine salt as a binder (inventive examples I-1 to I-3).

Table 1: Formulations for tableting, the amount of ingredients is given in %w/w

| Ingredients (%w/w) | C-1 | C-2 | I-1 | I-2 | I-3 |
|---|---|---|---|---|---|
| Metformin hydrochloride | 100 | 100 | 50 | 70 | 70 |
| Omega-3 lysine salt (spray granulated) | 0 | 0 | 50 | 30 | 0 |
| Omega-3 lysine salt (spray dried) | 0 | 0 | 0 | 0 | 30 |
| Total | 100 | 100 | 100 | 100 | 100 |

Metformin hydrochloride was mixed with omega-3 lysine salt powder as mentioned in table 1. Compression was carried out using 12 mm circular biconvex punch, average weight of the tablet was 555 mg. Friability test and tablets breaking forces were determined as per method describe in United States Pharmacopeia (USP<1216>). A maximum weight loss from the samples of not more than 1.0% is considered acceptable for most products. A weight loss of less than 3% is considered acceptable for specialized dosage forms like MUPS.

Table 2: Results for tableting trials

| Parameter | C-1 | C-2 | I-1 | I-2 | I-3 |
|---|---|---|---|---|---|
| Av. compress. force (kN) | 4.50 | 11.50 | 4.50 | 11.50 | 11.50 |
| Av. tablet break. force (N) | 5.00 | 5.00 | 50.50 | 50.50 | 52.50 |
| Av. ejection force (N) | 106.50 | 106.50 | 104.50 | 107.50 | 105.50 |
| Friability 100 rotations (%) | > 10 | > 10 | 0.7 | 0.8 | 0.7 |
| Binding Parameter (BP) | 1.11 | 0.43 | 11.22 | 4.39 | 4.57 |
| Tablet thickness (mm) | 5.5-5.6 | 5.2-5.3 | 5.5-5.6 | 5.3-5.4 | 5.3-5.4 |
| Disintegration time in water (min) | 1 | 3 | 75 | 30 | 30 |

Metformin tablets prepared without omega-3 lysine salt compressed at low compression force (4.5 kN) and compressed at high compression force (11.5 kN) showed capping and breaking after friability test and hence failed the friability test. While tablets prepared with 30% to 50% w/w omega-3 lysine salt showed good strength and passes both the friability test (<1%) and has high BP-value of more than 2.

## B) Omega-3 amino acid salt as binder in Ashwagandha tablets

For the comparative examples (C-3 to C-6), Ashwagandha powder was compressed without addition of a binder under different conditions. Compression was carried out using 12 mm circular biconvex punch, average weight of the tablet was 555 mg.

Table 3: Results for tableting trials

| Parameter | C-3 | C-4 | C-5 | C-6 |
|---|---|---|---|---|
| Av. compress. force (kN) | 3.50 | 4.50 | 9.50 | 15.50 |
| Av. tablet break. force (N) | 2.50 | 2.50 | 13.00 | 33.00 |
| Av. ejection force (N) | 110.5 | 110.5 | 105.5 | 111 |
| Friability 100 rotations (%) | > 10 | > 10 | > 10 | > 10 |
| Binding Parameter (BP) | 0.71 | 0.56 | 1.37 | 2.13 |
| Tablet thickness (mm) | 6.6-6.7 | 6.5-6.6 | 5.8-6.0 | 5.6-5.7 |
| Disintegration time in water (min) | 0.3 | 0.3 | 0.3 | 0.5 |

As shown in table 3, at a compression force up to 15.50 kN (up to 21 kN was tested), the tablets had a high friability of > 10% when compressed without addition of omega-3 lysine salt. This shows that the Ashwagandha powder is not compressible alone at different compression force tested.

Table 4: Formulations for tableting, the amount of ingredients is given in %w/w

| Ingredients (%w/w) | I-4 | I-5 | I-6 | I-7 | I-8 | I-9 | I-10 | I-11 |
|---|---|---|---|---|---|---|---|---|
| Ashwagandha Powder | 50 | 50 | 50 | 70 | 70 | 90 | 90 | 90 |
| Omega-3 lysine salt | 50 | 50 | 49.5 | 30 | 30 | 10 | 0 | 10 |
| Omega-3 arginine salt | 0 | 0 | 0 | 0 | 0 | 0 | 10 | 0 |
| Magnesium stearate | 0 | 0 | 0.5 | 0 | 0 | 0 | 0 | 0 |
| Total | 100 | 100 | 100 | 100 | 100 | 100 | 100 | 100 |

Table 5: Results for tableting trials

| Parameter | I-4 | I-5 | I-6 | I-7 | I-8 | I-9 | I-10 | I-11 |
|---|---|---|---|---|---|---|---|---|
| Av. compress. force (kN) | 3.50 | 24.00 | 3.50 | 4.50 | 15.50 | 10.50 | 10.50 | 10.50 |
| Av. tablet break. force (N) | 121.5 | 146.0 | 93.0 | 86.00 | 112.5 | 51.50 | 25.00 | 33.00 |
| Av. ejection force (N) | 111.5 | 104 | 114.8 | 113.5 | 104.5 | 104.5 | 108.9 | 110.5 |
| Friability 100 rotations (%) | 0 | 0.1 | 0 | 0 | 0.1 | 0.2 | 0 | 0 |
| Binding Parameter (BP) | 34.71 | 6.08 | 26.57 | 19.11 | 7.26 | 4.90 | 2.38 | 3.14 |
| Tablet thickness (mm) | 5.6-5.8 | 5.5-5.6 | 5.7-5.8 | 5.7-5.9 | 5.5-5.6 | 5.8-5.9 | 5.7-5.8 | 5.8-6.0 |
| Disintegration time in water (min) | 60 | 60 | 50 | 6 | 10 | 0.5 | 0.5 | 0.5 |

For the inventive examples (I-4 to I-10) Ashwagandha Powder was mixed with omega-3 salt powder as mentioned in table 4. For inventive example I-11 Ashwagandha powder and omega-3-lysine salt were mixed together and wet granulation was carried out using water. Wet granulated mass was dried in tray dryer for 6 hours at 50°C. After drying the prepared granules were sifted through 30# sieve. Compression was carried out using 12 mm circular biconvex punch, average weight of the tablet was 555 mg. The results are summarized in table 5.

As shown in table 5, tablets prepared with different concentrations of omega-3 salts prepared by either direct compression or wet granulation had good tablet strength and did not show any sign of breaking or capping during friability test. All inventive examples (I-4 to I-11) had a friability of < 1% and a BP value of more than 2.

## C) Omega-3 amino acid salt as binder in lactose tablets

Lactose tablets were prepared without addition of a binder (comparative example C-7) and with addition of an omega-3 lysine salt as a binder (inventive examples I-12 to I-13).

Table 6: Formulations for tableting, the amount of ingredients is given in %w/w

| Ingredients (%w/w) | C-7 | I-12 | I-13 |
|---|---|---|---|
| Lactose monohydrate | 100 | 90 | 50 |
| Omega-3 lysine salt | 0 | 10 | 50 |
| Total | 100 | 100 | 100 |

Lactose monohydrate was mixed with omega-3 lysine salt powder as mentioned in table 6. Compression was carried out using 12 mm circular biconvex punch, average weight of the tablet was 555 mg. Friability test and tablets breaking forces were determined as per method describe in United States Pharmacopeia (USP). The results are summarized in table 7.

Table 7: Results for tableting trials

| Parameter | C-7 | I-12 | I-13 |
|---|---|---|---|
| Av. compress. force (kN) | 11.50 | 11.00 | 11.00 |
| Av. tablet break. force (N) | 9.00 | 50.00 | 60.50 |
| Av. ejection force (N) | 101 | 101.5 | 101.5 |
| Friability 100 rotations (%) | > 5 | 0.90 | 0.60 |
| Binding Parameter (BP) | 0.78 | 4.55 | 5.50 |
| Tablet thickness (mm) | 5.1-5.2 | 5.1-5.2 | 5.1-5.2 |
| Disintegration time in water (min) | 3 | 9 | 31 |

Lactose tablets prepared without omega-3 lysine salt showed capping after friability testing and hence failed the test. While the tablets prepared with omega-3 lysine salt as binder had good strength and friability of < 1% and a high BP value of more than 2.

### D) Omega-3 amino acid salt as binder and diluent in Pantoprazole MUPS tablets

Pentoprazole MUPS tablets were prepared without addition of a binder (comparative example C-8) and with addition of an omega-3 lysine salt as a binder (inventive examples I-14 to I-15).

Table 8: Formulations for tableting, the amount of ingredients is given in %w/w

| Ingredients (%w/w) | C-8 | I-14 | I-15 |
|---|---|---|---|
| Pantoprazole pellets coated with EUDRAGIT FL 30 D (18/25 mesh size) | 42.81 | 42.81 | 42.81 |
| MCC 101 granules (#20 passed) | 10.81 | - | - |
| MCC 102 | 3 | - | - |
| MCC 200 | 29.53 | 22.19 | 22.19 |
| Ceolus KG 802 | 10.31 | - | - |
| Ac-Di-sol SD-711 | 2.26 | 5.0 | 5.0 |
| Aerosil 200 | 0.71 | - | - |
| Sod. Stearyl fumarate | 0.57 | - | - |
| Omega-3 lysine salt | - | 30 | 30 |
| Total | 100 | 100 | 100 |

All ingredients as listed in table 8 were mixed and compressed using 17.1 X 8.6 mm caplet shat punch, average weight of the tablet was 725 mg. Friability test and tablets breaking forces were determined as per method describe in United States Pharmacopeia (USP). The results are summarized in table 9.
MUPS formulation prepared without omega-3 lysine salt (example C-8) and compressed at compression force of 5.5 kN showed color change of pellets from white to brown in 0.1N HCl after 2 hours, although having a high BP value. When analyzed, there was 16.2% drug release in 0.1N HCl after 2 hours. This color change and higher release in 0.1N HCl is not desirable because it leads to degradation of the API due to loss of integrity (breaking/cracking) of coated pellets in the MUPS. While the MUPS formulation prepared with 30% w/w of omega-3 lysine salt compressed up to compression force of 7.5 kN did not show any color change of pellets after 2 hours exposure to 0.1N HCl and the drug release was only 3.6% indicating that the integrity of coated pellets maintained due to presence of omega-3 lysine salt. Moreover, those MUPS formulations had a high BP value of more than 2.

Table 9: Results for tableting trials

| Parameter | C-8 | I-14 | I-15 |
|---|---|---|---|
| Av. compress. force (kN) | 5.50 | 2.95 | 7.50 |
| Av. tablet break. force (N) | 85.00 | 59.60 | 62.00 |
| Av. ejection force (N) | 110.5 | 110.5 | 110.5 |
| Friability 100 rotations (%) | 0.80 | 2.90 | 1.40 |
| Binding Parameter (BP) | 15.45 | 20.20 | 8.27 |
| Tablet thickness (mm) | 6.9-7.0 | 6.9-7.0 | 6.5-6.6 |
| Visual observation of integrity after 2 hours of exposure to 0.1N HCl | Pellets turn brown, indicat. loss of integrity | No change in color | No change in color |
| Release of drug after 2 hours in 0.1N HCl (%, limit: < 10%) | 16.2 | - | 3.6 |

### E) Omega-3 fatty acid salts (magnesium and ornithine) as binder in Metformin tablets

Metformin tablets were prepared without addition of a binder (comparative examples C-1 and C-2) and with addition of an omega-3 magnesium salt and omega-3 ornithine salt as a binder (inventive examples I-16 and I-17).

Table 10: Formulations for tableting, the amount of ingredients is given in %w/w

| Ingredients (%w/w) | I-16 | I-17 |
|---|---|---|
| Metformin hydrochloride | 70 | 70 |
| Omega-3 magnesium salt | 30 | 0 |
| Omega-3 ornithine salt | 0 | 30 |
| Total | 100 | 100 |

Metformin hydrochloride was mixed with omega-3 fatty acid salt powder as mentioned in table 10. Compression was carried out using 12 mm circular biconvex punch, average weight of the tablet was 555 mg. Friability test and tablets breaking forces were determined as per method describe in United States Pharmacopeia (USP). A maximum weight loss from the samples of not more than 1.0% is considered acceptable for most products. A weight loss of less than 3% is considered acceptable for specialized dosage forms like MUPS.

The results for the tableting trials are summarized in table 11. In the comparative example C-1 and C-2, Metformin tablets were prepared without omega-3 fatty acid salts, both compressed at low compression force (4.5 kN) and compressed at high compression force (11.5 kN) showed capping and breaking after friability test and hence failed the friability test (a shown in tables 1 and 2). While the tablets prepared with 30% w/w omega-3 magnesium salt and omega-3 ornithine salt showed good strength and both passed the friability test (<1%). For both formulations, the BP value was more than 2.

Table 11: Results for tableting trials

| Parameter | I-16 | I-17 |
|---|---|---|
| Average Compression force(kN) | 11.50 | 11.50 |
| Average Tablet Breaking Force(N) | 34.91 | 34.18 |

(continued)

| Parameter | I-16 | I-17 |
|---|---|---|
| Average Ejection Force(N) | 112.5 | 113 |
| Friability at 100 rotations (%) | 0.9 | 0.09 |
| Binding Parameter (BP) | 3.04 | 2.97 |
| Tablet thickness(mm) | 5.7-5.8 | 5.4-5.5 |
| Disintegration time water(min) | 5 | 25 |

**Claims**

1. A method of preparing a solid dosage form, comprising the steps of:

   a. preparing a binder consisting of at least one polyunsaturated fatty acid salt;
   b. adding the binder and further ingredients to a mixer;
   c. optionally carrying out one or more of the following steps: granulation, drying and sizing,
   d. blending the contents of the mixer;
   e. compressing or slugging the blended contents to produce a solid dosage form,
   wherein the binding parameter (BP) for the solid dosage form is at least 2 and is determined by:

$$BP = \frac{H}{C}$$

   wherein H is the tablet breaking force in Newton (N) and C is the compression force in kilo Newton (kN).

2. The method according to claim 1, wherein the friability of the solid dosage form is 5% or less, preferably 3% or less, more preferably 1% or less.

3. The method according to any one of the preceding claims, wherein the mean particle size of the binder before mixing is between 2 $\mu$m and 600 $\mu$m.

4. The method according to any one of the preceding claims, wherein the binder is prepared by

   - admixing aqueous, aqueous-alcoholic or alcoholic solutions of a composition comprising at least one polyunsaturated omega-3 fatty acid or omega-6 fatty acid component and a composition containing a basic organic acid selected from lysine, arginine, ornithine, choline or at least counter ion selected from magnesium (Mg$^{2+}$) and potassium (K$^+$) and mixtures of the same, and
   - subjecting resulting admixture to spray drying conditions or an extruder-based process subsequently, thus forming a solid product composition comprising at least one salt of a cation derived from the basic amino acid or magnesium (Mg$^{2+}$) or potassium (K$^+$) with an anion derived from a polyunsaturated omega-3 fatty acid or omega-6 fatty acid.

5. The method according to any one of the preceding claims, wherein the spray drying conditions comprise a pure spray drying or a spray granulation process, or continuous spray granulation.

6. Use of a preparation comprising at least one polyunsaturated fatty acid salt comprising at least one omega-3 or omega-6 fatty acid salt selected from eicosapentaenoic acid (EPA), docosahexaenoic acid (DHA), arachidonic acid (ARA), alpha linolenic acid, stearidonic acid, eicosatetraenoic acid, docosapentaenoic acid, linoleic acid, $\gamma$-linolenic acid as binder in tableting applications for compression of solid components.

7. Preparation according to claim 6, wherein the omega-3 fatty acid component is selected from EPA or DHA.

8. Preparation according to claim 6 or 7, wherein the omega-3 or omega-6 fatty acid salts have an organic counter ion selected from lysine, arginine, ornithine, choline or magnesium (Mg$^{2+}$) or potassium (K$^+$) and mixtures of the same.

**Patentansprüche**

1. Verfahren zur Herstellung einer festen Dosierungsform, umfassend die Schritte:

   a. Herstellen eines Bindemittels, das aus mindestens einem Salz einer mehrfach ungesättigten Fettsäure besteht;
   b. Zugeben des Bindemittels und weiterer Bestandteile zu einem Mischer;
   c. gegebenenfalls Durchführen eines oder mehrerer der folgenden Schritte: Granulation, Trocknen und Klassieren,
   d. Mischen des Inhalts des Mischers;
   e. Komprimieren oder Slugging des gemischten Inhalts zum Erhalt einer festen Dosierungsform,
   wobei der Bindungsparameter (BP) für die feste Dosierungsform mindestens 2 beträgt und bestimmt wird durch:

   $$BP = \frac{H}{C}$$

   wobei H die Tablettenbrechkraft in Newton (N) ist und C die Kompressionskraft in Kilonewton (kN) ist.

2. Verfahren nach Anspruch 1, wobei die Friabilität der festen Dosierungsform 5 % oder weniger, vorzugsweise 3 % oder weniger, weiter bevorzugt 1 % oder weniger, beträgt.

3. Verfahren nach einem der vorhergehenden Ansprüche, wobei die mittlere Partikelgröße des Bindemittels vor dem Mischen zwischen 2 $\mu$m und 600 $\mu$m liegt.

4. Verfahren nach einem der vorhergehenden Ansprüche, wobei das Bindemittel hergestellt wird, indem man

   - wässrige, wässrig-alkoholische oder alkoholische Lösungen einer Zusammensetzung, die mindestens eine mehrfach ungesättigte Omega-3-Fettsäure- oder Omega-6-Fettsäurekomponente umfasst, und einer Zusammensetzung, die eine basische organische Säure, die aus Lysin, Arginin, Ornithin, Cholin ausgewählt ist, oder mindestens ein Gegenion, das aus Magnesium (Mg$^{2+}$) und Kalium (K$^+$) und Mischungen davon ausgewählt ist, enthält, mischt und
   - anschließend die erhaltene Mischung Sprühtrocknungsbedingungen oder einem extruderbasierten Verfahren unterwirft, wodurch eine feste Produktzusammensetzung gebildet wird, die mindestens ein Salz eines von der basischen Aminosäure oder Magnesium (Mg$^{2+}$) oder Kalium (K$^+$) abgeleiteten Kations mit einem von einer mehrfach ungesättigten Omega-3-Fettsäure oder Omega-6-Fettsäure abgeleiteten Anion umfasst.

5. Verfahren nach einem der vorhergehenden Ansprüche, wobei die Sprühtrocknungsbedingungen eine reine Sprühtrocknung oder einen Sprühgranulationsprozess oder eine kontinuierliche Sprühgranulation umfassen.

6. Verwendung einer Zubereitung, die mindestens ein Salz einer mehrfach ungesättigten Fettsäure umfasst, das mindestens ein Omega-3- oder Omega-6-Fettsäuresalz, das aus Eicosapentaensäure (EPA), Docosahexaensäure (DHA), Arachidonsäure (ARA), alpha-Linolensäure, Stearidonsäure, Eicosatetraensäure, Docosapentaensäure, Linolsäure, $\gamma$-Linolensäure ausgewählt ist, umfasst, als Bindemittel bei Tablettieranwendungen zur Komprimierung von festen Komponenten.

7. Zubereitung nach Anspruch 6, wobei die Omega-3-Fettsäurekomponente aus EPA oder DHA ausgewählt ist.

8. Zubereitung nach Anspruch 6 oder 7, wobei die Omega-3- oder Omega-6-Fettsäuresalze ein organisches Gegenion aufweisen, das aus Lysin, Arginin, Ornithin, Cholin oder Magnesium (Mg$^{2+}$) oder Kalium (K$^+$) und Mischungen davon ausgewählt ist.

**Revendications**

1. Procédé de préparation d'une forme posologique solide, comprenant les étapes de :

   a. préparation d'un liant constitué d'au moins un sel d'acide gras polyinsaturé ;
   b. ajout du liant et d'autres ingrédients à un mélangeur ;

c. éventuellement réalisation d'une ou plusieurs des étapes suivantes : granulation, séchage et calibrage,
d. mélangeage des contenus du mélangeur ;
e. compression ou mise en pastilles des contenus mélangés pour produire une forme posologique solide,
dans lequel le paramètre de liaison (BP) pour la forme posologique solide est d'au moins 2 et est déterminé par :

$$BP = \frac{H}{C}$$

dans lequel H est la force de rupture du comprimé en Newton (N) et C est la force de compression en kilo Newton (kN).

2. Procédé selon la revendication 1, dans lequel la friabilité de la forme posologique solide est de 5 % ou moins, de préférence de 3 % ou moins, plus préférablement de 1 % ou moins.

3. Procédé selon l'une quelconque des revendications précédentes, dans lequel la taille moyenne des particules du liant avant mélange est comprise entre 2 $\mu$m et 600 $\mu$m.

4. Procédé selon l'une quelconque des revendications précédentes, dans lequel le liant est préparé par

- mélange de solutions aqueuses, hydroalcooliques ou alcooliques d'une composition comprenant au moins un composant acide gras oméga-3 ou acide gras oméga-6 polyinsaturé et d'une composition contenant un acide organique basique choisi parmi la lysine, l'arginine, l'ornithine, la choline ou au moins un contre-ion choisi parmi le magnésium ($Mg^{2+}$) et le potassium ($K^+$) et leurs mélanges, et
- soumission du mélange résultant à des conditions de séchage par pulvérisation ou à un procédé basé sur une extrudeuse, formant ainsi une composition de produit solide comprenant au moins un sel d'un cation dérivé de l'acide aminé basique ou du magnésium ($Mg^{2+}$) ou du potassium ($K^+$) avec un anion dérivé d'un acide gras oméga-3 ou d'un acide gras oméga-6 polyinsaturé.

5. Procédé selon l'une quelconque des revendications précédentes, dans lequel les conditions de séchage par pulvérisation comprennent un séchage par pulvérisation pur ou un procédé de granulation par pulvérisation ou une granulation par pulvérisation continue.

6. Utilisation d'une préparation comprenant au moins un sel d'acide gras polyinsaturé comprenant au moins un sel d'acide gras oméga-3 ou oméga-6 choisi parmi l'acide eicosapentaénoïque (EPA), l'acide docosahexaénoïque (DHA), l'acide arachidonique (ARA), l'acide alpha linolénique, l'acide stéaridonique, l'acide eicosatétraénoïque, l'acide docosapentaénoïque, l'acide linoléique, l'acide $\gamma$-linolénique comme liant dans des applications de mise en comprimé pour la compression de composants solides.

7. Préparation selon la revendication 6, dans laquelle le composant acide gras oméga-3 est choisi parmi EPA ou DHA.

8. Préparation selon la revendication 6 ou 7, dans laquelle les sels d'acides gras oméga-3 ou oméga-6 ont un contre-ion choisi parmi la lysine, l'arginine, l'ornithine, la choline ou le magnésium ($Mg^{2+}$) ou le potassium ($K^+$) et leurs mélanges.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2019034698 A1
- US 2014274987 A1
- WO 2017202942 A1
- US 2010272792 A1
- WO 2016102323 A1
- EP 0734373 B1
- WO 2016102316 A1
- WO 2017202935 A1

**Non-patent literature cited in the description**

- *A flexible technology for modified-release drugs: Multiple-unit pellet system (MUPS)*
- **ABDUL et al.** *Journal of Controlled Release*, 2019, vol. 147, 2-16